# FASCICULE DE BREVET EUROPEEN

(11) **EP 1 827 260 B1**
(45) Date de publication et mention de la délivrance du brevet: **17.09.2008**
(21) Numéro de dépôt: 05824460.9
(22) Date de dépôt: 07.12.2005
(51) Int. Cl.: A61B 17/34, A61B 10/00, A61B 17/16

(54) **TROCART PERFORANT**
PERFORIERENDER TROKAR
PERFORATING TROCAR

(30) Priorité: 08.12.2004 US 634104 P
(43) Date de publication de la demande: 05.09.2007
(73) Titulaire: Laurane Medical, F-78730 St. Arnoult En Yvelines (FR)
(72) Inventeur: MASSEGLIA, Thierry, F-83210 La Farlede (FR); FUMEX, Laurent, F-78730 St Arnoult En Yvelines (FR)
(74) Mandataire: L'Helgoualch, Jean
(86) Numéro de dépôt international: PCT/FR2005/003068
(87) Numéro de publication internationale: WO 2006/061514

(56) Documents cités:
- WO-A-03/020140
- DE-U1- 8 634 674
- US-A- 5 810 826
- US-A1- 2002 042 581

## Description

La présente invention concerne le domaine des instruments utilisables en chirurgie et en radiologie interventionnelle, et plus particulièrement un trocart perforant utilisable notamment dans le domaine de la biopsie osseuse, de la cimentoplastie des regions squelettiques, et du traitement des lésions osseuses par photocoagulation ou thermocoagulation.

On connaît divers types de trocarts qui sont des instruments chirurgicaux utilisés pour accéder à des cavités naturelles ou pathologiques, pour effectuer des ponctions ou pour introduire des substances, par exemple pour effectuer des coelioscopies intestinales ou gynécologiques, ou encore pour réaliser des opérations endoscopiques, notamment arthroscopiques, qui permettent de réduire fortement la période postopératoire du patient par rapport aux opérations de chirurgie ouverte. Un trocart est généralement constitué d'un tube creux, encore appelé canule ou gaine, dans lequel peut coulisser une tige ou obturateur dont l'extrémité distale, débouchant à l'extrémité distale du tube, est en forme de pointe pour faciliter la pénétration dans les tissus.

Ainsi, le brevet FR 2.697.150 décrit un trocart destiné à la coelioscopie comprenant un tube dans lequel s'engage une tige creuse munie d'une pointe à facettes évidées et susceptible de contenir un dispositif de protection de la pointe. La demande de brevet WO 03020140 décrit un trocart conçu pour ne requérir qu'une force minimale d'insertion dans les tissus afin de réduire autant que possible les dommages causés par la pénétration, ledit trocart comportant une pointe à l'extrémité distale de l'obturateur s'adaptant parfaitement dans le prolongement de l'ouverture distale de la canule qui présente une certaine flexibilité. Un tel trocart est essentiellement destiné à être introduit dans des tissus mous. Un autre type de trocart est décrit dans la demande de brevet WO 03045260 suivant laquelle un obturateur perforant peut coulisser dans la canule du trocart afin de percer la paroi abdominale d'un patient au cours d'une opération laparoscopique consistant à insuffler un gaz dans la cavité abdominale pour la distendre et faciliter l'opération. Le trocart conforme à cette technique n'est cependant pas conçu pour perforer une paroi dure comme celle d'un os.

On connaît aussi quelques types de trocarts perforants disponibles sur le marché des instruments chirurgicaux, comportant une pointe associée à un tube à bord distal coupant afin de permettre la pénétration dans un os relativement dur mais l'efficacité des trocarts de ce type n'est pas toujours satisfaisante, en particulier dans le cas d'os de forte dureté résistant aux outils de coupe ordinaires. Ainsi, le brevet US 5.810.826 décrit un trocart perforant comportant une tige à extrémité en forme de mèche à pointe excentrée, guidée par un tube. Quand la tige entre en contact avec l'os, sa rotation provoque la formation d'un trou de diamètre plus large que celui de la tige, en raison de la position excentrée de la pointe, tandis que le tube est maintenu immobile. L'utilisation de ce trocart est diffficile car l'opérateur doit maintenir le tube d'une main et faire tourner la tige de l'autre main.

La présente invention a pour objet un trocart perforant procurant une bonne efficacité de perforation, ainsi qu'une grande facilité et une bonne sécurité de manipulation.

L'invention a encore pour objet un trocart perforant permettant d'effectuer, dans de bonnes conditions d'efficacité et de fiabilité, des opérations telles que des biopsies osseuses, des interventions de vertébroplastie et des traitements de lésions.

Le trocart suivant la présente invention est du type comportant un tube rigide dans lequel peut coulisser une tige à pointe distale perforante, et il se distingue en ce que la zone de la pointe distale de la tige a la forme d'une mèche perforante pouvant tourner sur son axe tandis que l'extrémité distale du tube est divisée en au moins deux segments à bord coupant hélicoïdal. Conformément à l'invention, chaque segment hélicoïdal correspond à un angle inférieur ou égal à 180° autour de l'axe du tube.

Les segments à bord coupant hélicoïdal sont de préférence identiques ou présentent des profils voisins ou similaires. Par segments à profils voisins on entend des segments sensiblement identiques ou ne différant que par des détails mineurs. Par segments à profils similaires, on entend des segments de même forme mais présentant des dimensions différentes.

Suivant une forme avantageuse de réalisation de l'invention, l'extrémité distale du tube est divisée en deux segments symétriques suivant l'axe du tube, et le bord coupant de chaque partie forme une rampe hélicoïdale inclinée de 1 à 45°, et de préférence de 10 à 30°, par rapport au plan perpendiculaire à l'axe. Les bords de ces parties, selon l'épaisseur du tube, peuvent être affûtés pour améliorer l'efficacité de la coupe, par exemple en prévoyant une section en biseau.

Suivant une forme préférentielle de réalisation, les parties à bord hélicoïdal de l'extrémité distale du tube sont séparées par une encoche découpée dans la paroi du tube, la profondeur de l'encoche, depuis le bord inférieur de la rampe hélicoïdale jusqu'au fond de l'encoche, étant comprise entre 0,5 et 5 fois, et de préférence entre 1 et 2 fois le diamètre intérieur du tube.

Conformément à une autre caractéristique de l'invention, la zone de la pointe distale de la tige comporte au moins deux arêtes coupantes s'étendant depuis un point situé de préférence sur l'axe, jusqu'à la périphérie de la pointe. Suivant une forme préférentielle de réalisation, ces arêtes coupantes sont disposées symétriquement par rapport à l'axe de la tige, et leurs profils peuvent être identiques ou différents les uns des autres. Ainsi par exemple, deux arêtes coupantes peuvent être disposées de part et d'autre de l'axe de la tige et décentrées par rapport à lui. Suivant une variante conforme à l'invention, les arêtes coupantes s'étendent depuis un point légèrement excentré par rapport à l'axe.

Suivant une forme avantageuse de réalisation de l'invention, une partie au moins du corps de la tige a une forme de mèche perforante à deux goujures hélicoïdales. Les surfaces d'attaque de la pointe distale délimitées par les arêtes coupantes présentent une forme concave rejoignant chaque goujure, facilitant la perforation d'une surface dure, telle que celle d'un os, lorsque la tige est appliquée contre une telle surface et qu'un mouvement de rotation lui est appliqué.

La tige à pointe en forme de mèche décrite ci-dessus peut être retirée du tube après perforation de l'os, pour être remplacée, par exemple, par une aiguille ou canule à biopsie ou par une sonde à ultrasons ou des fibres optiques.

Le trocart de l'invention peut ainsi être utilisé non seulement en biopsie osseuse, mais aussi en cimentoplastie pour les régions squelettiques, en particulier en vertébroplastie pour l'injection d'un ciment de comblement osseux, ou encore pour le traitement de lésions osseuses par photocoagulation au moyen d'un laser à fibre optique, ou par thermocoagulation au moyen d'un appareil à radiofréquences ou à ultrasons.

A titre d'exemple, dans le cas d'une biopsie de lésion osseuse à effectuer derrière un os dur, le praticien, après avoir procédé à une anesthésie locale suivant les techniques classiques, introduit le trocart comportant le tube et une tige à extrémité distale en pointe, à travers la peau et les tissus mous du patient jusqu'à venir en contact avec l'os. Une butée de réglage, mobile sur le tube du trocart, permet d'assurer le réglage de la profondeur d'insertion en fonction de la distance suivant laquelle la perforation de l'os doit être faite. La tige est ensuite retirée tout en laissant le tube en place, son extrémité distale étant contre la paroi de l'os. La tige perforante est ensuite insérée dans la gaine jusqu'au verrouillage de sa poignée, sa pointe étant alors en contact avec l'os, et un mouvement de rotation est alors appliqué à l'ensemble constitué par le tube et la tige perforante pour forer l'os. La biopsie est ensuite effectuée en remplaçant la tige perforante par un instrument approprié, par exemple une aiguille à biopsie.

La structure du trocart de l'invention présente l'avantage d'une grande simplicité d'utilisation puisqu'il peut être mis en place et manipulé d'une seule main grâce à la coopération entre la gaine à bords coupants et la tige à pointe perforante qui sont solidaires lors du mouvement de perforation par rotation.

La simplicité de la structure du trocart de l'invention permet de proposer à l'utilisateur un ensemble cohérent, ou kit, constitué de plusieurs trocarts, tubes, tiges pleines ou creuses et aiguilles perforantes ou d'extraction, cet ensemble étant adapté à l'utilisation en chirurgie, et plus particulièrement à la réalisation de biopsies osseuses, par exemple pour des lésions plus ou moins profondes.

D'autres caractéristiques et avantages de la présente invention apparaîtront dans la description suivante, relative à une forme préférentielle de réalisation.

Le trocart comprend un tube cylindrique creux dans lequel peut coulisser et pivoter une tige. Le trocart comprend encore une poignée permettant sa manipulation par l'utilisateur, comportant deux parties, une partie intérieure solidaire du tube et une partie extérieure solidaire de la tige. Les deux parties de la poignée peuvent être déplacées l'une par rapport à l'autre afin de déplacer la tige dans le tube du trocart, par coulissement en tirant sur la partie extérieure de la poignée et en l'écartant de la partie intérieure, ou par pivotement en faisant tourner la partie extérieure par rapport à la partie intérieure de la poignée.

Le trocart comprend encore une butée dont la position sur le tube est réglable par l'intermédiaire d'une vis.

Le tube comporte deux encoches à section rectangulaire. Ces deux encoches divisent l'extrémité du tube en deux lèvres à arête coupante hélicoïdale, disposées symétriquement par rapport à l'axe du tube.

L'extrémité distale de la tige a la forme d'une mèche perforante, débordant au-delà de l'extrémité du tube lorsqu'elle est insérée totalement dans le tube, de telle sorte que les deux parties de la poignée sont alors jointives.

A la pointe du trocart, les positions respectives des deux lèvres sont symétriques par rapport à l'axe, et les deux arêtes coupantes ont des profils différents l'un de l'autre. L'inclinaison de l'arête coupante des lèvres par rapport au plan perpendiculaire à l'axe de la tige est d'environ 15°.

Les deux encoches facilitent l'évacuation des débris d'os lors de la perforation au moyen des lèvres coupantes dont l'action se conjugue avec celle de la pointe de la tige lorsque l'utilisateur la fait pivoter sur son axe dans le tube.

La pointe de la tige a une forme de mèche perforante à double goujure hélicoïdale à découpe terminale formant une arête coupante sensiblement sur l'axe de la tige, de telle sorte que la rotation de la tige dans le sens rétrograde (sens des aiguilles d'une montre) lorsque la pointe de la tige est contre une paroi osseuse, favorise la perforation de ladite paroi osseuse. Les surfaces d'attaque des arêtes coupantes présentent une forme concave délimitée sur un côté par les arêtes coupantes et sur les autres côtés par l'extrémité des goujures hélicoïdales.

## Revendications

1. Trocart perforant, en particulier pour biopsie osseuse, du type comportant un tube rigide et une tige à pointe distale perforante pouvant coulisser dans le tube, ladite pointe distale de la tige possédant une zone ayant la forme d'une mèche perforante pouvant tourner sur son axe, **caractérisé en ce que** l'extrémité distale du tube est divisée en au moins deux segments, à bord coupant hélicoïdal.

2. Trocart perforant selon la revendication 1, **caractérisé en ce que** chaque segment hélicoïdal correspond à un angle inférieur ou égal à 180° autour de l'axe du tube.

3. Trocart perforant selon la revendication 2, **caractérisé en ce que** l'extrémité distale du tube est divisée en deux segments symétriques suivant l'axe du tube.

4. Trocart selon la revendication 3, **caractérisé en ce que** le bord coupant de chaque segment forme une rampe hélicoïdale inclinée de 1 à 45° par rapport au plan perpendiculaire à l'axe.

5. Trocart selon la revendication 4, **caractérisé en ce que** le bord de chaque partie forme une rampe hélicoïdale inclinée de 10 à 30°,

6. Trocart selon la revendication 1, **caractérisé en ce que** les parties à bord hélicoïdal de l'extrémité distale du tube sont séparées par une encoche découpée dans la paroi du tube.

7. Trocart selon la revendication 6, **caractérisé en ce que** la profondeur de l'encoche, depuis le bord inférieur de la rampe hélicoïdale jusqu'au fond de l'encoche, est comprise entre 0,5 et 5 fois le diamètre intérieur du tube.

8. Trocart selon la revendication 1, **caractérisé en ce que** la zone de la pointe distale de la tige comporte au moins deux arêtes coupantes s'étendant depuis un point situé sur l'axe, jusqu'à la périphérie de la pointe.

9. Trocart selon la revendication 8, **caractérisé en ce que** la zone de la pointe distale de la tige comporte deux arêtes coupantes disposées de part et d'autre de l'axe de la tige et décentrées par rapport à lui.

10. Trocart selon la revendication 1, **caractérisé en ce que** la zone distale de la tige a une forme de mèche perforante à deux goujures hélicoïdales, et les surfaces d'attaque de la pointe distale délimitées par les arêtes coupantes présentent une forme concave rejoignant chaque goujure.

## Claims

1. Perforating trocar, in particular for bone biopsy, of the type comprising a rigid tube and a rod (3) with a perforating distal tip capable of sliding in the tube, said distal tip of the rod having a zone having the form of a perforating drill-bit that is able to turn on its axis, **characterized in that** the distal end of the tube is divided into at least two segments with a helical cutting edge.

2. Perforating trocar according to Claim 1, **characterized in that** each helical segment corresponds to an angle of less than or equal to 180° about the axis of the tube.

3. Perforating trocar according to Claim 2, **characterized in that** the distal end of the tube is divided into two symmetrical segments along the axis of the tube.

4. Trocar according to Claim 3, **characterized in that** the cutting edge of each segment forms a helical ramp inclined by 1 to 45° with respect to the plane perpendicular to the axis.

5. Trocar according to Claim 4, **characterized in that** the edge of each part forms a helical ramp inclined by 10 to 30°.

6. Trocar according to Claim 1, **characterized in that** the helical edge parts of the distal end of the tube are separated by a recess cut out in the wall of the tube

7. Trocar according to Claim 6, **characterized in that** the depth of the recess, from the lower edge of the helical ramp to the bottom of the recess, is between 0.5 and 5 times the internal diameter of the tube

8. Trocar according to Claim 1, **characterized in that** the zone of the distal tip of the rod comprises at least two cutting ridges that extend from a point situated on the axis to the periphery of the tip.

9. Trocar according to Claim 8, **characterized in that** the zone of the distal tip of the rod comprises two cutting ridges arranged on either side of the axis of the rod and off-centered relative to the latter.

10. Trocar according to Claim 1, **characterized in that** the distal zone of the rod has the form of a perforating drill-bit with two helical flutes, and the attacking surfaces of the distal tip, which are delimited by the cutting ridges, have a concave shape joining each flute

## Patentansprüche

1. Perforierender Trokar, insbesondere für eine Knochenbiopsie, vom Typ, der ein starres Rohr und einen Schaft mit einer distalen perforierenden Spitze umfasst, die in dem Rohr gleiten kann, wobei die distale Spitze des Schafts einen Bereich aufweist, der die Form eines perforierenden Bohrers aufweist, der sich um seine eigene Achse drehen kann, **dadurch gekennzeichnet, dass** das distale Ende des Rohrs in zumindest zwei schraubenförmige Schneidkanten-Segmente geteilt ist.

2. Perforierender Trokar nach Anspruch 1, **dadurch gekennzeichnet, dass** jedes schraubenförmige Segment einem Winkel von kleiner gleich 180° um die Achse des Rohrs entspricht.

3. Perforierender Trokar nach Anspruch 2, **dadurch gekennzeichnet, dass** das distale Ende des Rohrs in zwei in Bezug auf die Achse des Rohrs symmetrische Segmente geteilt ist.

4. Trokar nach Anspruch 3, **dadurch gekennzeichnet, dass** die Schneidkante jedes Segments eine schraubenförmige Rampe bildet, die in Bezug auf die Ebene, die im rechten Winkel auf die Achse steht, in einem Winkel von 1 bis 45° geneigt ist.

5. Trokar nach Anspruch 4, **dadurch gekennzeichnet, dass** die Wand jedes Teils eine schraubenförmige Rampe bildet, die in einem Winkel von 10 bis 30° geneigt ist.

6. Trokar nach Anspruch 1, **dadurch gekennzeichnet, dass** die Teile mit schraubenförmiger Kante des distalen Endes des Rohrs durch eine in der Rohrwand ausgeschnittene Kerbe getrennt sind.

7. Trokar nach Anspruch 6, **dadurch gekennzeichnet, dass** die Tiefe der Kerbe vom unteren Rand der schraubenförmigen Rampe bis zum Grund der Kerbe dem 0,5- bis 5-Fachen des Innendurchmessers des Rohrs entspricht.

8. Trokar nach Anspruch 1, **dadurch gekennzeichnet, dass** der Bereich der distalen Spitze des Schafts zumindest zwei Schneidkanten umfasst, die sich von einem Punkt auf der Achse bis zum Umfang der Spitze erstrecken.

9. Trokar nach Anspruch 8, **dadurch gekennzeichnet, dass** der Bereich der distalen Spitze des Schafts zwei Schneidkanten umfasst, die auf der einen und anderen Seite der Achse des Schafts und in Bezug auf diese dezentriert angeordnet sind.

10. Trokar nach Anspruch 1, **dadurch gekennzeichnet, dass** der distale Bereich des Schafts die Form eines perforierenden Bohrers mit zwei schraubenförmigen Nuten aufweist und dass die Angriffsflächen der distalen Spitze, die durch die Schneidkanten begrenzt sind, eine konkave Form aufweisen, die an jede Nute angrenzt.
